# EUROPÄISCHE PATENTANMELDUNG

(11) **EP 1 586 539 A1**
(43) Veröffentlichungstag der Anmeldung: **19.10.2005**
(21) Anmeldenummer: 04008805.6
(22) Anmeldetag: 13.04.2004
(51) Int. Cl.: C02F 1/32, B01D 53/00, B01J 19/12

(54) **Vorrichtung zur Behandlung eines flüssigen oder gasförmigen Mediums mittels UV-Strahlen**

(71) Anmelder: Araiza, Rafael, 6318 Walchwil (CH)
(72) Erfinder: Ariza, Rafael, 6318 Walchwil (CH); Hartig, Heinz, 81373 München (DE)

(57) **Zusammenfassung**

Vorrichtung zur Behandlung eines flüssigen oder gasförmigen Mediums, insbesondere Wasser oder Luft, mittels UV-Strahlen, umfassend: eine UV-Strahlungsquelle (24) mit einer axialen Längserstreckung und einer dazu im Wesentlichen senkrechten, insbesondere radialen Abstrahlungsrichtung (R); und mehrere Schichten von in Abstrahlungsrichtung (R) übereinander angeordneten, aufeinanderfolgenden, durchstrahlbaren Behandlungskammern (K1 - K4), die von der UV-Strahlungsquelle (24) und voneinander jeweils durch eine transparente, UVdurchlässige Trennschicht (T1 - T4) getrennt sind, und die, beginnend bei einer der UV-Strahlungsquelle (24) in Abstrahlungsrichtung nächstliegenden, ersten Behandlungskammer (K1), jeweils einen entlang der Längserstreckung der UV-Strahlungsquelle (24) verlaufenden Durchströmungskanal für das Medium bilden, der in die jeweils nachfolgende, von der UV-Strahlungsquelle (24) in Abstrahlungsrichtung (R) weiter entfernte Behandlungskammer (K2, K3, K4) mündet (26).

## Beschreibung

### TECHNISCHES GEBIET

Die vorliegende Erfindung betrifft eine Vorrichtung zur Behandlung eines flüssigen oder gasförmigen Mediums mittels ultravioletter Strahlen (UV-Strahlen). Ferner betrifft die Erfindung eine besonders geeignete Verwendung dieser Vorrichtung.

### STAND DER TECHNIK

Als UV-Strahlung bezeichnet man die elektromagnetische Strahlung, die zwischen der sichtbaren Grenze von kurzwelligem Licht und Röntgenstrahlung angesiedelt ist und in einem Wellenlängenbereich von ca. 100 nm bis 400 nm liegt. Man unterscheidet folgenden Bereiche: UVA-trahlung: 400 - 315 nm; UVB-Strahlung: 315 - 280 nm; UVC-Strahlung: ca. 280 - 200 nm; und VUV-Strahlung: ca. < 180 nm. Vorrichtungen zur Behandlung eines flüssigen oder gasförmigen Mediums mittels UV-Strahlen werden zum Beispiel zur UV-Entkeimung oder zur UV-Oxidation eingesetzt.

Bei der UV-Entkeimung wird in der Regel UVC-Strahlung verwendet, welche Mikroorganismen wie Bakterien, Hefen und Pilze durch Schädigung der DNS (Desoxiribonucleinsäure) wirksam abgetötet. Eine Wellenlänge der UV-Strahlung in einem Bereich von ca. 260 nm bis 280 nm, insbesondere bei ca. 254 nm, erzielt hierbei eine besonders hohe Entkeimungswirkung, da das Absorbtionsspektrum der DNS bei ca. 254 nm ein Maximum besitzt. Durch die hohe Absorption der DNS in diesem UV-Strahlungsbereich wird eine fotochemische Reaktion ausgelöst, die in den bestrahlten Mikroorganismen eine Unterbrechung der genetischen Information zur Zellvermehrung und für den Stoffwechsel bewirkt. Die Mikroorganismen werden auf diese Weise inaktiviert und unschädlich gemacht. Eine Zugabe von Chemikalien ist bei der UV-Entkeimung nicht erforderlich.

Bei Wellenlängen unter 230nm ist die Energie der UV-Strahlung bereits ausreichend, um chemische Bindungen aufzubrechen. UV-Strahlung mit Wellenlängen kleiner 200 nm beinhaltet ionisierende Strahlen, die fotochemische Oxidationsprozesse auslösen, die zur Reinigung von Gasen oder Flüssigkeiten, wie z.B. Abwässern, genutzt werden können, die mit organischen Schadstoffen, wie z.B. Pflanzenschutzmitteln, Hormonen, Dioxinen, Medikamentresten, etc. kontaminiert sind. Bei dieser UV-Oxidation werden die Moleküle der organischen Substanzen oxidiert und zu ungiftigen Verbindungen abgebaut. Dieses Verfahren wird auch als photochemische Nassverbrennung bezeichnet. Für industrielle Anwendungen wird dem Abwasser noch ein separates Oxidationsmittel (z.B. H₂O₂, Ozon) beigegeben. Durch die UV-Strahlung werden die oxidierbaren Substanzen im Abwasser mineralisiert. Zusätzlich wird das Oxidationsmittel in hoch reaktive Radikale gespalten. Diese Radikale tragen ebenfalls zur vollständigen Oxidation der unerwünschten Abwasserinhaltstoffe bei.

Aus der EP 0 470 518 A1 ist eine in Form eines fotochemischen Durchflussreaktors ausgestaltete Vorrichtung zur Behandlung eines flüssigen oder gasförmigen Mediums mittels UV-Strahlen bekannt. Diese zur UV-Oxidation verwendete Vorrichtung arbeitet vorzugsweise unter Verwendung eines zusätzlich zugeführten Oxidationsmittels. Die Vorrichtung umfasst eine UV-Strahlungsquelle mit einer axialen Längserstreckung und einer dazu im Wesentlichen senkrechten, radialen Abstrahlungsrichtung. Ferner besitzt die Vorrichtung eine einzelne Behandlungskammern in Gestalt eines langen Edelstahlbehälters, in welchem die UV-Strahlungsquelle angeordnet ist. Die Innenwand des Edelstahlbehälters ist poliert und dient als Reflektor für die UV-Strahlung. An der Innenwand des Edelstahlbehälters sind Verwirbelungselemente angebracht, welche das durchströmende Medium verwirbeln und vermischen und in den Wirkungsbereich der UV-Strahlungsquelle leiten sollen.

Zur Erzielung einer hinreichenden Entkeimungs- oder Oxidationswirkungsgrades sowie zur Erreichung eines ausreichenden Durchsatzes müssen konventionelle Vorrichtungen zur Behandlung eines flüssigen oder gasförmigen Mediums mittels UV-Strahlen eine erhebliche Baugröße aufweisen. Diese Baugröße erfordert nicht nur einen erheblichen Platzbedarf, sondern bedingt auch, dass die betreffende Vorrichtung nur mit relativ geringen Drücken arbeiten kann, da andernfalls Festigkeitsprobleme auftreten. Darüber hinaus ist bei vorbekannten Vorrichtungen eine sehr hohe Leistung der UV-Strahlungsquelle erforderlich. Bei konventionellen Vorrichtungen, die zur UV-Entkeimung verwendet werden, hat es sich femer gezeigt, dass oftmals ein Teil der Mikroorganismen in dem zu behandelnden Medium nicht vollständig inaktiviert wird. Darüber hinaus ist festzustellen, dass selbst bei einer vollständigen Inaktivierung der Mikroorganismen durch Zerstörung von deren DNS die Zellhülle der Mikroorganismen sowie deren Zusammensetzung weitgehend intakt bleibt. Dies kann bei Personen, die Unverträglichkeiten gegen bestimmte Proteine oder chemische Zusammensetzungen aufweisen, nachteilig sein. Im Falle einer unvollständigen UV-Oxidation wiederum können organische Schadstoffe in dem Medium verbleiben, was ebenfalls nicht erstrebenswert ist. Ferner wäre es wünschenswert, bei der UV-Oxidation möglichst weitgehend auf zusätzlich beizufügende Oxidationsmittel verzichten zu können.

### DARSTELLUNG DER ERFINDUNG

Der Erfindung liegt die Aufgabe beziehungsweise das technische Problem zugrunde, eine einfache und effektive Vorrichtung zur Behandlung eines flüssigen oder gasförmigen Mediums mittels UV-Strahlen zu schaffen, welche die dem Stand der Technik anhaftenden Nachteile möglichst vermeidet. Gemäß einem weiteren Aspekt dieser Aufgabe soll eine besonders geeignete Verwendung für eine solche Vorrichtung aufgezeigt werden.

Diese Aufgabe wird gemäß einem ersten Aspekt gelöst durch eine erfindungsgemäße Vorrichtung mit den Merkmalen des Anspruchs 1.

Diese Vorrichtung zur Behandlung eines flüssigen oder gasförmigen Mediums, insbesondere Wasser oder Luft, mittels UV-Strahlen, umfasst: eine UV-Strahlungsquelle mit einer axialen Längserstreckung und einer dazu im Wesentlichen senkrechten, insbesondere radialen Abstrahlungsrichtung; und mehrere (d.h. mindestens zwei) Schichten von in Abstrahlungsrichtung übereinander angeordneten, aufeinanderfolgenden, durchstrahlbaren Behandlungskammern, die von der UV-Strahlungsquelle und voneinander jeweils durch eine transparente, UV-durchlässige Trennschicht getrennt sind, und die, beginnend bei einer der UV-Strahlungsquelle in Abstrahlungsrichtung nächstliegenden, ersten Behandlungskammer, jeweils einen entlang der Längserstreckung der UV-Strahlungsquelle verlaufenden Durchströmungskanal für das Medium bilden, der in die jeweils nachfolgende, von der UV-Strahlungsquelle in Abstrahlungsrichtung weiter entfernte Behandlungskammer mündet.

Die erfindungsgemäße Vorrichtung arbeitet im Durchflussverfahren. Die UV-Strahlungsquelle emittiert vorzugsweise UV-Strahlen im UVC und/oder VUV-Bereich. Die UV-Strahlungsquelle kann eine einzige oder mehrere UV-Einzelstrahlungsquellen umfassen, die an einer gemeinsamen Stelle oder auch an unterschiedlichen Stellen der Vorrichtung angeordnet sind. Falls mehrere UV-Einzelstrahlungsquellen Anwendung finden, so ist es möglich, dass diese sowohl gleiche als auch unterschiedliche UV-Strahlungspektren aufweisen. Beispielsweise kann dann eine UV-Einzelstrahlungsquellen im UVC- und die andere und VUV-Bereich strahlen. Je nach konkretem Anwendungsfall sind als UV-Strahlungsquelle Nieder-, Mittel- und Hochdruckstrahler einsetzbar. Die Anzahl der Schichten von Behandlungskammern kann insbesondere in Abhängigkeit der UV-Strahlungsquelle, des zu behandelnden Mediums und seiner Verunreinigungen variieren. Ferner können die Abmessungen und Geometrien der jeweiligen Behandlungskammern, insbesondere ihre Dicke in Strahlungsrichtung bzw. ihre Strömungsquerschnitte gleich oder unterschiedlich sein. In Verbindung mit dem jeweils zu behandelnden Medium ist dadurch eine gewünschte Strömungsdynamik sowie eine optimale Ausnutzung der Eindringtiefe der UV-Strahlung oder eines bestimmten Spektralbereichs in das Medium und ein bestimmte Absorptions- und Reaktionsverhalten des Mediums erreichbar. Auch die transparenten, UV-durchlässigen Trennschichten oder Teilbereiche davon können gleiche oder unterschiedliche Eigenschaften, insbesondere UV-Transmissionseigenschaften, und Abmessungen bzw. Dicken aufweisen.

Die erfindungsgemäße Vorrichtung ist gegenüber konventionellen gattungsgemäßen Vorrichtungen vergleichsweise klein, kompakt und mit einem niedrigen Bauvolumen ausführbar und benötigt nur einen geringen Platzbedarf. Dennoch ist sie sehr robust. Die kompakte Bauweise ermöglicht es auch, mit höheren Drücken als beim Stand der Technik zu arbeiten, wodurch der Durchsatz des Mediums erhöht bzw. mit einer kleineren Vorrichtung ein gleicher Durchsatz wie bei einer größeren vorbekannten Vorrichtung erzielt werden kann. Während vorbekannte Vorrichtungen beispielsweise mit ca. 1,5 bar arbeiten, so ist die erfindungsgemäße Vorrichtung z.B. ohne Weiteres mit 4,5 bar betreibbar. Durch die geschichtete bzw. mehrfach geschichtete Anordnung der Behandlungskammern lässt sich trotz der geringen Baugröße die Verweilzeit des zu behandelnden Mediums und der darin enthaltenen Verunreinigungen im Wirkungsbereich der UV-Strahlungsquelle erheblich erhöhen. Infolge der erfindungsgemäßen Bauweise wird hierbei der Strahlungsbereich der UV-Strahlungsquelle optimal genutzt und die Einwirkungsdauer der UV-Strahlen auf kleinstem Raum maximiert, da das zu behandelnde Medium während eines einzigen Durchlaufs durch die Vorrichtung mehrfach durch den Wirkungsbereich der UV-Strahlungsquelle geleitet wird. Die erfindungsgemäße Vorrichtung bietet aufgrund ihrer speziellen Konstruktionsweise darüber hinaus weitere Möglichkeit zur Verlängerung der Verweilzeit, auf die weiter unten noch näher eingegangen werden wird.

Aufgrund der erhöhten Verweilzeit des zu behandelnden Mediums im Strahlungsbereich der UV-Strahlungsquelle besitzt die erfindungsgemäße Vorrichtung gegenüber vorbekannten Lösungen einen erheblich verbesserten Entkeimungs- und/oder Oxidationswirkungsgrad. In dem Medium enthaltene Mikroorganismen somit zuverlässig deaktivierbar. Und etwaige organische Schadstoffe lassen sich vollständig zu ungiftigen Verbindungen oxidieren bzw. abbauen.

Darüber hinaus ist es jedoch ein besonderer Vorteil der erfindungsgemäßen Vorrichtung, dass sich mit dieser innerhalb eines einzelnen Gerätes ein kombinierter UV-Entkeimungs- und UV-Oxidationsprozess (nachfolgend kurz kombinierter UV-Prozess genannt) durchführen lässt. Diese Möglichkeit ergibt sich primär durch die spezielle Schichtanordnung der aufeinanderfolgenden, von der UV-Strahlung durchstrahlbaren Behandlungskammern in Verbindung mit der Erhöhung der Verweilzeit des Medium und der Einwirkdauer der UV-Strahlung auf das Medium und dessen Verunreinigungen. Bei geeigneter Wahl des Spektralbereichs der UV-Strahlungsquelle wird bei dem kombinierten UV-Prozess das Medium sowohl durch Schädigung der DNS der enthaltenen Mikroorganismen entkeimt, als auch die molekulare Zusammensetzung der verbleibenden inaktivierten Mikroorganismen oder Viren durch photochemische Oxidation (z.B. im Rahmen einer Nassverbrennung) zerstört und vollständig abgebaut. Es ergibt sich somit ein sehr hoher Reinigungsgrad.

Bei der konventionellen UV-Entkeimung ggf. auftretende Unverträglichkeiten gegen bestimmte Proteine oder chemische Zusammensetzungen von inaktivierten aber physikalisch noch im Medium existenten Mikroorganismen oder -resten sind somit wirkungsvoll vermeidbar. Je nach Art der verwendeten UV-Strahlungsquelle bzw. der Anordnung ihrer UV-Einzelstrahlungsquelle kann bei dem so erzielbaren kombinierten UV-Prozess die UV-Entkeimung und UV-Oxidation gewissermaßen fließend ineinander über gehen oder aber auch sequentiell in aufeinanderfolgenden Behandlungskammern ausgeführt werden.

Mit der erfindungsgemäßen Vorrichtung ist somit ein Verfahren ausführbar, welches einem natürlichen, durch die UV-Strahlung der Sonne erzielbaren Prozess gleicht, bei dem letztendlich ungiftige, nicht mehr aktive Stoffe bzw. Verbindungen zurückbleiben, die ihrerseits wieder in den Biokreislauf zurückgeführt werden können.

Obwohl bei dem mit der erfindungsgemäßen Vorrichtung realisierbaren kombinierten UV-Prozess grundsätzlich separate Oxidationsmittel bei der UV-Oxidation eingesetzt werden könnten, ist dies aufgrund des erreichbaren hohen Wirkungsgrades in der Regel nicht erforderlich. Eine Gefährdung durch die Nutzung derartiger Chemikalien ist damit vermeidbar. Infolge der optimalen Ausnutzung der UV-Strahlung benötigt die erfindungsgemäße Vorrichtung auch nur eine UV-Strahlungsquelle mit einer vergleichsweise geringen Leistung. Die erfindungsgemäße Vorrichtung arbeitet ergo mit hohem Wirkungsgrad, niedrigen Betriebskosten, geringem Wartungsaufwand und folglich hoher Wirtschaftlichkeit.

Weitere bevorzugte und vorteilhafte Ausgestaltungsmerkmale der erfindungsgemäßen Vorrichtung sind Gegenstand der Unteransprüche 2 bis 15.

Die der Erfindung zugrunde liegende Aufgabe wird gemäß einem zweiten Aspekt gelöst durch die erfindungsgemäße Verwendung nach Anspruch 16. Diese sieht eine Verwendung der Vorrichtung nach einem oder mehreren der Ansprüche 1 bis 15 zur kombinierten UV-Entkeimung und UV-Oxidation (oben kombinierter UV-Prozess genannt), insbesondere zur Wasseraufbereitung vor.

Durch die erfindungsgemäße Verwendung sind im Wesentlichen die gleichen Vorteile zur erreichen, die zuvor im Zusammenhang mit der erfindungsgemäßen Vorrichtung dargelegt wurden.

Bevorzugte Ausführungsbeispiele der Erfindung mit zusätzlichen Ausgestaltungsdetails und weiteren Vorteilen sind nachfolgend unter Bezugnahme auf die beigefügten Zeichnungen näher beschrieben und erläutert.

### KURZE BESCHREIBUNG DER ZEICHNUNGEN

Es zeigt:
- Fig. 1: ein schematisches Blockschaltbild einer erfindungsgemäßen Vorrichtung gemäß einer ersten Ausführungsform;;
- Fig. 2: eine schematische Längsschnittansicht durch eine wesentliche Komponenten der erfindungsgemäßen Vorrichtung gemäß der ersten Ausführungsform;
- Fig. 3: eine schematische Querschnittsansicht entlang der Linie A-A in Fig. 2;
- Fig. 4: eine schematische Querschnittsansicht entlang der Linie B-B in Fig. 2;
- Fig. 5: eine schematische Querschnittsansicht entlang der Linie C-C in Fig. 2;
- Fig. 6: eine schematische Draufsicht auf die Vorrichtung von Fig. 2 mit Blickrichtung gemäß dem Pfeil D in Fig. 1;
- Fig. 7: eine schematische Längsschnittansicht durch eine erfindungsgemäßen Vorrichtung gemäß einer zweiten Ausführungsform;
- Fig. 8: eine schematische Querschnittansicht durch eine erfindungsgemäßen Vorrichtung gemäß einer dritten Ausführungsform; und
- Fig. 9: eine schematische Querschnittansicht durch eine erfindungsgemäßen Vorrichtung gemäß einer vierten Ausführungsform.

### DARSTELLUNG VON BEVORZUGTEN AUSFÜHRUNGSBEISPIELEN

In der nachfolgenden Beschreibung und in den Figuren werden zur Vermeidung von Wiederholungen gleiche Bauteile und Komponenten auch mit gleichen Bezugszeichen gekennzeichnet, sofern keine weitere Differenzierung erforderlich oder sinnvoll ist.

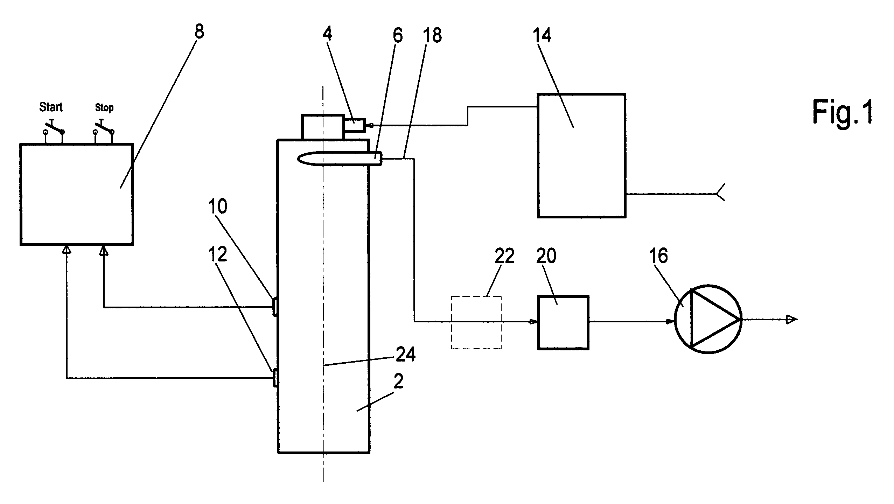

Fig. 1 zeigt ein schematisches Blockschaltbild einer erfindungsgemäßen Vorrichtung zur Behandlung eines flüssigen oder gasförmigen Mediums mittels UV-Strahlen, gemäß einer ersten Ausführungsform. Die Vorrichtung umfasst als wesentliche Komponenten einen fotochemischen Reaktor 2 mit einem Einspeisungskanal 4 für das zu behandelnde flüssige oder gasförmige Medium und einem Auslasskanal 6 für das behandelte Medium. Als zu behandelndes Medium wird im vorliegenden Ausführungsbeispiel Wasser verwendet, das durch Mikroorganismen bzw. Viren und organische Schadstoffe verunreinigt ist.

Der Reaktor 2 ist mit einer Kontrolleinrichtung 8 gekoppelt, die hier über zwei Sicherheits- und Kontrollsensoren 10, 12 (hier: ein optischer Sensor und ein Temperatursensor) verfügt. Die Vorrichtung besitzt des weiteren eine Vorfiltereinrichtung 14, die dem Einspeisungskanal 4 vorgeschaltet ist, sowie eine Pumpe 16, die in einer an den Auslasskanal 6 angeschlossenen Leitung 18 angeordnet ist. Zwischen dem Auslasskanal 6 und der Pumpe 16 ist ein Magnetventil 20 vorgesehen. In der Leitung 18 kann auch eine dem Auslasskanal 6 nachgeschaltete Filtereinrichtung 22 (z.B. ein Aktivkohlefilter) vorgesehen werden, welche zum Herausfiltem etwaiger Radikalenreste und/oder Ozonreste in dem behandelten Medium dient.

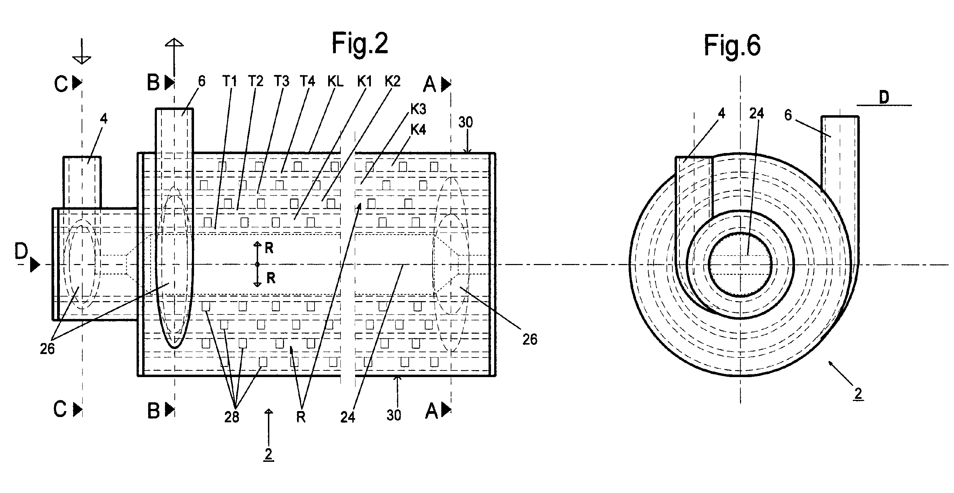

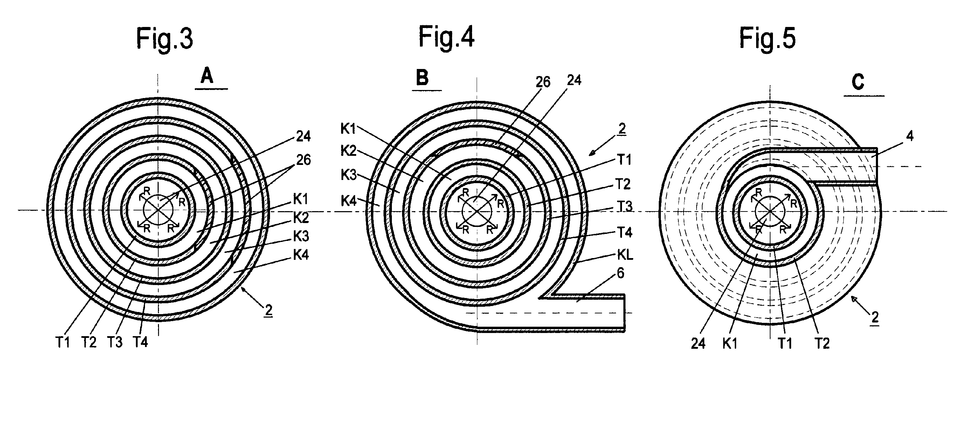

In Fig. 2 ist ein schematischer Längsschnittansicht durch den fotochemischen Durchflussreaktor 2 der erfindungsgemäßen Vorrichtung gemäß der ersten Ausführungsform dargestellt. Fig. 3 zeigt eine schematische Querschnittsansicht entlang der Linie A-A in Fig. 2. Die Vorrichtung bzw. der Reaktor 2 umfasst eine UV-Strahlungsquelle 24 mit einer axialen Längserstreckung und einer dazu im Wesentlichen senkrechten, insbesondere radialen Abstrahlungsrichtung R. Die UV-Strahlungsquelle 24 besitzt ein UV-Strahlungsspektrum von mindestens 180 nm bis zumindest 254 nm Wellenlänge. Im vorliegenden Fall liegt das UV-Strahlungsspektrum in einem Bereich von ca. 180nm bis ca. 260 nm.

Des weiteren besitzt die Vorrichtung mehrere Schichten von in Abstrahlungsrichtung übereinander angeordneten bzw. geschichteten, aufeinanderfolgenden, durchstrahlbaren Behandlungskammern K1 bis K4. Die Behandlungskammern K1 - K4 sind von der UV-Strahlungsquelle 24 und voneinander jeweils durch eine transparente, UV-durchlässige Trennschicht T1 bis T4 getrennt. Beginnend bei einer der UV-Strahlungsquelle 24 in Abstrahlungsrichtung R nächstliegenden, ersten Behandlungskammer K1, bilden die Behandlungskammern K1 - K4 jeweils einen entlang der Längserstreckung der UV-Strahlungsquelle 24 verlaufenden Durchströmungskanal für das Medium, der in die jeweils nachfolgende, von der UV-Strahlungsquelle 24 in Abstrahlungsrichtung R weiter entfernte Behandlungskammer mündet.

Wie in den Fig. 2 und 3 angedeutet ist, besitzt der Reaktor 2 im vorliegenden Beispiel vier Behandlungskammern K1, K2, K3, K4, deren Länge im Wesentlichen der axialen Länge der UV-Strahlungsquelle 24 entspricht. Die Behandlungskammern K1 - K4 sind in radialer Richtung konzentrisch um die UV-Strahlungsquelle 24 herum angeordnet. Der Querschnitt der Behandlungskammern K1 - K4 ist jeweils kreisringförmig. Die Behandlungskammern K5 - K5 sind durch fünf Trennschichten voneinander separiert. Zumindest die ausgehend von der UV-Strahlungsquelle 24 ersten vier Trennschichten T1 - T4 sind transparent und für UV-Strahlung durchlässig.

Bei der ersten Ausführungsform sind ausgehend von der UV-Strahlungsquelle 24 in Abstrahlungsrichtung R sowohl die erste Trennschicht T1, die zwischen der UV-Strahlungsquelle 24 und der ersten Behandlungskammer K1 angeordnet ist und der UV-Strahlungsquelle 24 am nächsten liegt, als auch die in radialer Richtung nachfolgende zweite Trennschicht T2 aus einem ersten Trennschicht-Material hergestellt. Dieses erste Trennschicht-Material besitzt eine UV-Durchlässigkeit, die im Wesentlichen das gesamte UV-Strahlungsspektrum der UV-Strahlungsquelle 24 einschließlich eines ersten Teilspektrums mit Wellenlängen kleiner 200nm, also auch die UV-Strahlung mit ca. 180 nm durchlässt. Die ersten beiden Trennschichten T1, T2 sind in Form von Glasrohren ausgestaltet, die aus synthetischem Quarzglas als das erste Trennschicht-Material hergestellt sind.

Grundsätzlich könnte die erste Trennschicht T1 bzw. das erste Glasrohr auch eine transparente Hüllenwandung der UV-Strahlungsquelle 24 selbst sein, die für den genannten Spektralbereich durchlässig ist. Dies bietet sich z.B. dann an, wenn als UV-Strahlungsquelle 24 eine in der ersten Behandlungskammer K1 angeordnete UV-Tauchlampe verwendet wird.

Die in Abstrahlungsrichtung R gemessene Gesamtdicke der Teilschicht, die aus den ersten beiden Glasrohren T1, T2 und den ersten beiden Behandlungskammern K1, K2 gebildet wird, in welche die UV-Strahlung durch das synthetische Quarzglas eintritt, ist so gewählt, dass sie im Wesentlichen einer maximalen effektiven Eindringtiefe des ersten Teilspektrums der UV-Strahlung entspricht, die sich in Abhängigkeit der Absorptionsfähigkeit des zu behandelnden Wassers und der UV-Durchlässigkeit der ersten beiden Glasröhre T1, T2 für dieses erste Teilspektrum ergibt. Die gewährleistet eine optimale Ausnutzung der kurzwelligen, harten UV-Strahlung kleiner 200 nm zum Zwecke einer nachfolgend noch beschriebenen Ozon- bzw. Radikalenerzeugung. Eine größere Schichtdicke ist nicht sinnvoll, sondern ggf. sogar gefährlich, da dann Bereiche des zu behandelnden Wassers in Abstrahlrichtung R nicht mehr zuverlässig durchdrungen bzw. erfasst werden könnten.

Die radial weiter außen liegenden Trennschichten, d.h. hier zumindest die dritte und vierte Trennschicht T3, T4 in Abstrahlungsrichtung R der ersten und zweiten Trennschicht T1, T2 nachfolgen, sind aus einem zweiten Trennschicht-Material hergestellt. Dieses zweite Trennschicht-Material besitzt eine UV-Durchlässigkeit, die von dem UV-Strahlungsspektrum der UV-Strahlungsquelle 24 nur UV-Strahlung in einem zweiten Teilspektrum mit Wellenlängen größer gleich 200nm durchlässt. Die dritte und vierte Trennschicht T3, T4 sind ebenfalls in Form von Glasrohren ausgebildet, die natürlichem Quarzglas als das zweite Trennschicht-Material hergestellt sind.

Die erfindungsgemäße Vorrichtung verfügt des Weiteren über eine Verweilzeit-Verlängerungseinrichtung zum Verlängern der Verweilzeit des zu behandelnden Wassers und der darin enthaltenen Verunreinigungen bzw. Schadstoffe im Strahlungsbereich der UV-Strahlungsquelle 24. Die aufeinanderfolgenden Behandlungskammern K1 - K4 bilden hierbei einen Teil der Verweilzeit-Verlängerungseinrichtung. Denn das Wasser strömt im Betrieb der Vorrichtung zunächst durch den Einspeisungskanal 4 in die erste, radial innere Kammer K1, in dieser entlang der axialen Längserstreckung der UV-Strahlungsquelle 24 bis zu einer endseifigen Austrittsöffnung 26, die gleichzeitig eine endseitige Eintrittsöffnung 26 für die nächste, d.h. die zweite, radial nachfolgende Behandlungskammer K2 bildet. Hierbei wird das Wasser um ca. 180° umgelenkt, strömt wieder entlang der Längserstreckung der UV-Strahlungsquelle 24 und tritt in die dritte, radial weiter außen liegende Behandlungskammer K4, wobei es wiederum um 180° umgelenkt wird. Von der dritten Behandlungskammer K3 gelangt es auf analoge Art und Weise in die vierte, radial äußerste Kammer K4 und verlässt die Vorrichtung durch den Auslasskanal 6. Auf dem gesamten Strömungsweg durch die Behandlungskammern K1 - K4 ist das Wasser der UV-Strahlung ausgesetzt.

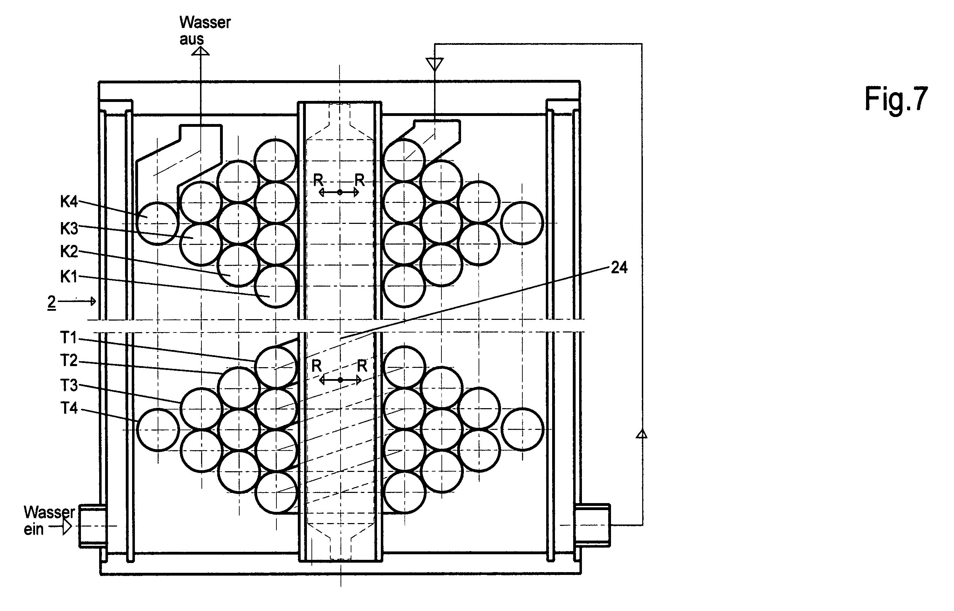

Die Verweilzeit-Verlängerungseinrichtung umfasst jedoch noch weitere Detailelemente, welche nachfolgend unter Bezugnahme auf die Fig. 4 bis 6 beschrieben werden. Hierbei zeigt Fig. 4 eine schematische Querschnittsansicht entlang der Linie B-B in Fig. 2; Fig. 5 eine schematische Querschnittsansicht entlang der Linie C-C in Fig. 2; und Fig. 6 eine schematische Draufsicht auf die Vorrichtung von Fig. 2 mit Blickrichtung gemäß dem Pfeil D in Fig. 1.

Wie in der Fig. 4 angedeutet ist, ist der Einspeisungskanal 4 als Teil der Verweilzeit-Verlängerungseinrichtung so ausgestaltet, dass er bezogen auf die radiale Abstrahlungsrichtung R bzw. die kreisringförmige Querschnittsform der ersten Behandlungskammern K1 im Wesentlichen tangential in die erste Behandlungskammer K1 mündet. Dem einzuspeisenden Wasser wird dadurch ein Drall verliehen, so dass es in der ersten Behandlungskammer K1 auf einer annähernd spiralförmigen Bahn um die UV-Strahlungsquelle 24 herum von der Einspeisungsöffnung 4 zu der Austrittsöffnung 26 der ersten Behandlungskammer K1 strömt. Die Austrittsöffnung 26 der ersten Behandlungskammer K1 bzw. die Eintrittsöffnung 26 der zweiten Behandlungskammer K2 ist z.B. schlitzförmig mit einem linsenartigen Schlitzquerschnitt ausgestaltet (vgl. auch Fig. 2 und 3), so dass das Wasser weitgehend ungehindert in Drallrichtung in die zweite Behandlungskammer K2 strömen kann. Dieser Vorgang wird durch die infolge des Dralls hervorgerufenen Fliehkräfte unterstützt. Die folgenden Austritts- bzw. Eintrittsöffnung 26 der jeweiligen radial nachfolgenden Behandlungskammern K3, K4 sind entsprechend konstruiert.

Auch der Auslasskanal 6 ist als Teil der Verweilzeit-Verlängerungseinrichtung ausgestaltet. Bezogen auf die radiale Abstrahlungsrichtung R bzw. die kreisringförmige Querschnittsform der Behandlungskammern K1 - K4 tritt er im Wesentlichen tangential und in Drallrichtung aus der letzten, der UV-Strahlungsquelle 24 in Abstrahlungsrichtung R am weitesten entfernten Behandlungskammer K4 heraus und trägt somit zur Aufrechterhaltung der spiralförmigen Strömung um die UV-Strahlungsquelle 24 bei.

Zusätzlich kann die Verweilzeit-Verlängerungseinrichtung Verwirbelungselemente 28 oder Turbulatoren aufweisen, die zum Beispiel in den Behandlungskammern K1 - K4, dem Einspeisungskanal 4, dem Auslasskanal 6, Eintritts- oder Austrittsöffnungen 26 zwischen aufeinanderfolgenden Behandlungskammern oder weiteren Zu- und Ableitungen angeordnet sind. Als Verwirbelungselemente 28 kommen z.B. noppenartige Erhöhungen, Vertiefungen, Schaufeln, Flügel, Zäune, rotierende oder schwingende Teile o.ä. auf den Wandungen der genannten Komponenten in Betracht. Sofern diese Verwirbelungselemente 28 in Verbindung mit dem besagten tangenfialen Einspeisungskanal 4 und Auslasskanal 6 verwendet werden, sollten sie zur Aufrechterhaltung der grundlegenden Drallrichtung beitragen. Die Verwirbelungselemente 28 können fest installiert oder aber auch an herausnehmbaren Einsätzen angeordnet sein, was Reinigungsarbeiten erleichtert. In der Fig. 2 sind Wirbel-Noppen angedeutet, die an den Glasrohren T1 - T4 angeordnet sind.

Die letzte, d.h. hier die vierte Behandlungskammer K4 besitzt an ihrer von der UV-Strahlungsquelle 24 in Abstrahlungsrichtung am weitesten entfernten Seite eine UV-Reflektionseinrichtung 30, welche die von der UV-trahlungsquelle 24 emittierte und bis zur äußeren Wandung KL durchgedrungene UV-Strahlung wieder zurückreflektiert und somit zu einem hohen Ausnutzungsgrad der UV-trahlung und einer intensiven Behandlung des Wassers beiträgt. Falls die äußere Wandung KL der letzten Behandlungskammer K4 für das UV-Licht undurchlässig ist, kann es sich bei der UV-Reflektionseinrichtung 30 z.B. um eine auf die Innenseite dieser Wandung KL aufgetragende Spiegeischicht oder eine stark polierte, reflektierende Oberfläche handeln. Ist die äußere Wandung KL für die UV-Strahlung hingegen durchlässig (z.B. aus natürlichem Quarzglas, so kann die UV-Reflektionseinrichtung 30 z.B. in Form einer um das Glas herum angebrachten Reflektions- oder Spiegelschicht oder dergleichen ausgeführt werden.

Es wird nun die Funktionsweise der erfindungsgemäßen Vorrichtung beschreiben werden.

Die Vorrichtung wird über die Kontrolleinrichtung 8 in Betrieb gesetzt und die UV-Strahlungsquelle 24 aktiviert. Nach einer vorgegebenen Einbrennzeit wird das Magnetventil 20 geöffnet und die Pumpe 16 betätigt. Kontaminiertes Wasser wird über die Vorfiltereinrichtung 14 in den Reaktor 2 und durch dessen Behandlungskammern K1 - K4 hindurch gesaugt, dort entkeimt und von chemischen Verunreinigungen befreit und fließt über den Auslasskanal 6, den eventuellen Nachfilter 22, das Magnetventil 20 und die Pumpe 16 aus.

Der optische Sensor 10 hat im Betrieb die Aufgabe, eine Trübung der Glaswände der Glasrohre T1 - T4 zu erfassen. Eine Trübung kann z.B. durch Kalkablagerungen und/oder durch trübes Wasser verursacht werden. Sie würde die Leistung des Reaktors einschränken. Bei einer definierten Trübung wird dies der Kontrolleinrichtung 8 über ein entsprechendes Sensorsignal gemeldet. Bei einer zu starken Trübung schaltet die Kontrolleinrichtung 8 die UV-Strahlungsquelle 24, das Magnetventil 20 und die Pumpe 16 ab und lässt einen emeuten Start nicht zu. Erst nach einer Reinigung oder Fehlerbeseitigung wird der Betrieb wieder freigegeben. Der Temperatursensor 12 hat die Aufgabe, den Reaktor vor Überhitzung zu schützen. Bei einem eventuell blockiertem Wasserfluss, würde sich der Reaktor aufheizen und das darin befindliche Wasser eventuell kochen. Der Sensor 12 meldet solch einen gefährlichen Zustand an die Kontrolleinrichtung 8 und diese schaltet dann die UV-Strahlungsquelle 24, das Magnetventil 20 und die Pumpe 16 ab.

Der Reinigungsvorgang als solcher läuft in de erfindungsgemäßen Vorrichtung wie folgt ab:
Das schadstoffbelastete, mit Mikroorganismen bzw. Viren kontaminierte Wasser wird in der ersten und zweiten Behandlungskammer K1, K2 mit UV-Strahlung beaufschlagt, welches ein UV-Strahlungsspektrum mit Wellenlängen von sowohl kleiner als auch größer 200nm besitzt. Die UV-Strahlung größer 200 nm, insbesondere um 254 nm, bewirkt hierbei die UV-Entkeimung durch Zerstörung der DNS der im Wasser enthaltenen Mikroorganismen. Die Strahlung unterhalb 200nm, insbesondere um ca. 180 nm setzt die UV-Oxidation im Rahmen einer photochemischen Nassverbrennung in Gang.

Die Strahlung unter 200nm produziert aus dem im Wasser befindlichen molekularen Sauerstoff (O₂) Ozon (O₃). Dies erfolgt in der ersten und zweiten Kammer. Das Ozon wird durch die vorhandene UV-Strahlung oberhalb von 200nm in einzelne (single) Sauerstoffatome (O) zerlegt. Diese an sich schon hoch reaktiven Sauerstoffatome verbinden sich infolge der fotochemischen Reaktion mit Wasserstoffatomen (H+) zu OH-Molekülen. OH-Moleküle sind freie Radikale, welche im Wesentlichen alle Arten der zuvor beschriebenen organischen Schadstoffe, insbesondere die molekularen Reste oder Hüllen der deaktivierten Mikroorganismen, zerstört. OH-Radikale besitzen eine max. Lebensdauer von ca. 100 Millisekunden. Danach zerfallen sie wieder zu Wasserstoff und Sauerstoff und damit zu absolut ungiftigen Stoffen. Es bleiben bei einer photochemischen Reaktion keinerlei schädliche Substanzen zurück. Alle organischen Stoffe werden völlig oxidiert (verbrannt). Auch Stoffe wie z.B. Pflanzenschutzmittel (DDT, Atrazine usw.) oder Zwischenprodukte von chemischen Reaktionen werden zu ungiftigen Resten oxidiert.

Bei der erfindungsgemäßen Vorrichtung erfolgt die Radikalerzeugung in den ersten beiden Behandlungskammern K1, K2. Da das die Radikale enthaltene Wasser fortwährend weiterströmt, findet die Oxidationen ("der Verbrauch") der Radikale hingegen weitgehend in der dritten und vierten Behandlungskammer K3, K4 statt. Die oben beschriebene inhärente bzw. zusätzlich ausgestaltete Verweilzeit-Verlängerungsvorrichtung der erfindungsgemäßen Vorrichtung ermöglicht die Erzeugung einer hohen Radikalendichte und steigert durch die Verwirbelung des Wassers gleichzeitig die Treffsicherheit der OH-Radikale auf die zu zerstörenden Molekularstrukturen. Die zur photochemischen Nassverbrennung notwendige Verweilzeit wird mit der erfindungsgemäßen Konstruktion mehr als erfüllt. Die notwendige Verweilzeit zur Oxidation von Eiweißkörpem liegt z.B. bei ca. 100 mS. Die bei einer Strömungsgeschwindigkeit von z.B. 3,8l/min in dem Reaktor der erfindungsgemäßen Vorrichtung erzielbare max. Verweilzeit liegt bei ca. 6000 - 7000 mS.

Da das dritte und vierte Glasrohr T3, T4 aus normalem bzw. natürlichem Quarzglas hergestellt ist und UV-Strahlung unterhalb 200nm nicht durchlässt und die UV-Strahlung unterhalb 200nm zudem bereits in der ersten und zweiten Behandlungskammer K1, K2 im Wesentlichen vollständig absorbiert wurde, ist sie in der dritten und vierten Behandlungskammer K3, K4 nicht mehr wirksam. Für die dritte und vierte Behandlungskammer K3, K4 sind die Trennschichten T3, T4 aus normalem bzw. natürlichem Quarz, welches für UV-Strahlung oberhalb von 200nm durchlässig ist, daher völlig ausreichend. Aus der letzten Behandlungskammer K4 tritt das Wasser tangential aus und steht dann entkeimt und von organischen Schadstoffen und Rückständen von Mikroorganismen bzw. Viren befreit zur Verfügung.

In der erfindungsgemäßen Vorrichtung findet somit fortlaufend eine kombinierte UV-Entkeimung und UV-Oxidation statt (weiter oben als kombinierter UV-Prozess bezeichnet), welcher einen sehr hohen Reinigungsgrad des kontaminierten Wassers gewährleistet.

Nach der Behandlung im Reaktor 2 sollte das entkeimte und gereinigte Wasser zur Sicherheit über Aktivkohle geleitet werden, was in der Nachfiltereinrichtung 22 erfolgen kann. Aktivkohle stellt wieder einen natürlichen Redox-Wert her. Denn bedingt durch die OH-Radikale herrscht in den Behandlungskammern K1 - K4 des Reaktors 2 ein enorm hohes Redoxpotential von bis zu 2Volt. Dieses ist schädlich und muss wieder auf die natürlichen Verhältnisse (250 - 450 mV) eingestellt werden.

Fig. 7 zeigt eine schematische Längsschnittansicht durch eine erfindungsgemäßen Vorrichtung gemäß einer zweiten Ausführungsform. Bei dieser Variante erstrecken sich die jeweiligen, schichtweise angeordneten Behandlungskammern K1 - K4 jeweils spiralförmig um die UV-Strahlungsquelle 24 herum.

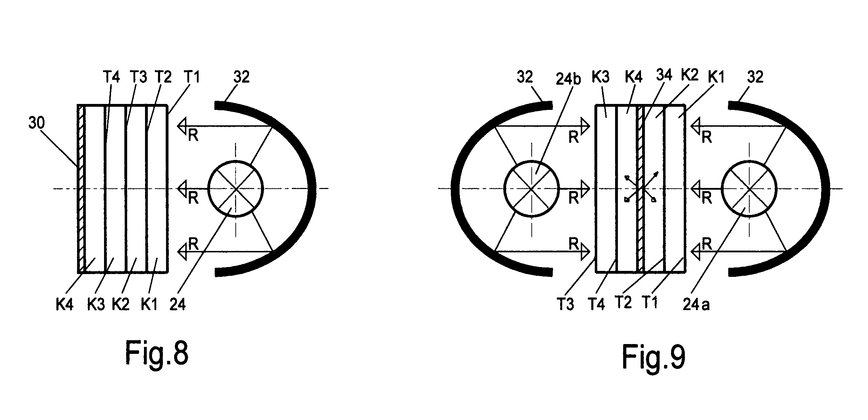

Fig. 8 zeigt eine schematische Querschnittansicht durch eine erfindungsgemäße Vorrichtung gemäß einer dritten Ausführungsform. Das Funktionsprinzip dieser Variante entspricht im Wesentlichen dem der ersten Ausführungsform, jedoch sind die schichtweise angeordneten Behandlungskammern K1 - K4 in einer plattenartigen bzw. kastenartigen Anordnung gruppiert, und die UV-Strahlungsquelle 24 ist seitlich dieser Anordnung platziert. Jede Behandlungskammer K1 - K4 besitzt eine meanderförmige Strömungsbahn (nicht gezeigt) für das zu behandelnde Medium. Für eine höhere UV-Lichtausbeute ist die Vorrichtung mit einer parabolischen Reflektoreinrichtung 32 ausgestattet, welche das von der UV-Strahlungsquelle 24 emittierte UV-Licht auf die geschichteten Behandlungskammern K1 - K4 bündelt.

In der Fig. 9 ist eine schematische Querschnittansicht durch eine efindungsgemäßen Vorrichtung gemäß einer vierten Ausführungsform dargestellt. Die Ausführungsform nach Fig. 9 ähnelt vom Prinzip her der nach Fig. 8, jedoch sind zwei UV-Einzelstrahlungsquellen 24a, 24b vorgesehen, welche die geschichteten Behandlungskammern K1 - K4 von zwei unterschiedlichen Seiten her durchstrahlen. Die erste UV-Einzelstrahlungsquelle 24a emittiert hierbei UV-Licht in einem Spektrum kleiner 200 nm und die zweite 24b in einem Spektrum größer 200 nm. Wird diesen UV-Einzelstrahlungsquelle 24a, 24b jeweils eine Gruppe von Behandlungskammern zugeordnet und zwischen diesen z.B. jeweils ein Filter oder eine trennende UV-Reflektionseinrichtung 34 angeordnet, so kann innerhalb der Vorrichtung die UV-Entkeimung und UV-Oxidation zeitlich und örtlich getrennt ausgeführt werden, z.B. erst nur die UV-Entkeimung und anschließend die UV-Oxidation. Als Filter können hierbei auch das erste oder zweite Material für die jeweiligen transparenten, UV-durchlässigen Trennschichten fungieren.

Die Erfindung ist nicht auf die obigen Ausführungsbeispiele beschränkt, die lediglich der allgemeinen Erläuterung des Kemgedankens der Erfindung dienen. Im Rahmen des Schutzumfangs kann die erfindungsgemäße Vorrichtung vielmehr auch andere als die oben konkret beschriebenen Ausgestaltungsformen annehmen. So können die Behandlungskammern beispielsweise auch transparente, UV-durchlässige Trennschichten aufweisen, die pro einzelner Trennschicht unterschiedliche Durchlässigkeiten bzw. Transmissionseigenschaften für unterschiedliche UV-Strahlungsspektren bzw. Teilspektren aufweisen. Obwohl in den obigen Beispielen natürliches oder synthetisches Quarzglas für die Trennschichten verwendet wurde, können grundsätzlich auch andere UV-durchlässige Materialien Anwendung finden, wenn diese die für jeweils benötigten Wellenlängenbereich der UV-Strahlung die oben beschriebenen Eigenschaften aufweisen. Je nach Anwendungsfall kann die Anzahl der Behandlungskammer variieren, die beträgt jedoch mindestens zwei.

Bezugszeichen in den Ansprüchen, der Beschreibung und den Zeichnungen dienen lediglich dem besseren Verständnis der Erfindung und sollen den Schutzumfang nicht einschränken.

### Bezugszeichenliste

Es bezeichnen:
- 2: Reaktor
- 4: Einspeisungskanal
- 6: Auslasskanal
- 8: Kontrolleinrichtung
- 10: Sensor
- 12: Sensor
- 14: Vorfiltereinrichtung
- 16: Pumpe
- 18: Leitung
- 20: Magnetventil
- 22: Nachgeschaltete Filtereinrichtung
- 24: UV-Strahlungsquelle
- 26: Austrittsöffnung / Eintrittsöffnung
- 28: Verwirbelungselemente
- 30: UV-Reflektionseinrichtung
- 32: UV-Reflektoreinrichtung
- 34: Trennende UV-Refelktionseinrichtung

- K1 - K4: Behandlungskammern
- KL: Letzte bzw. äußerste Wandung
- R: Abstrahlrichtung von 24
- T1, T2: Transparente, UV-durchlässige Trennschicht aus synthetischem Quarzglas
- T3, T4: Transparente, UV-durchlässige Trennschicht aus natürlichem Quarzglas

## Patentansprüche

1. Vorrichtung zur Behandlung eines flüssigen oder gasförmigen Mediums, insbesondere Wasser oder Luft, mittels UV-Strahlen, umfassend:
- eine UV-Strahlungsquelle (24; 24a, 24b) mit einer axialen Längserstreckung und einer dazu im Wesentlichen senkrechten, insbesondere radialen Abstrahlungsrichtung (R); und
- mehrere Schichten von in Abstrahlungsrichtung (R) übereinander angeordneten, aufeinanderfolgenden, durchstrahlbaren Behandlungskammern (K1 - K4),
die
von der UV-Strahlungsquelle (24; 24a, 24b) und voneinander jeweils durch eine transparente, UV-durchlässige Trennschicht (T1 - T4) getrennt sind,
und die,
beginnend bei einer der UV-Strahlungsquelle (24) in Abstrahlungsrichtung nächstliegenden, ersten Behandlungskammer (K1),
jeweils einen entlang der Längserstreckung der UV-Strahlungsquelle (24; 24a, 24b) verlaufenden Durchströmungskanal für das Medium bilden, der in die jeweils nachfolgende, von der UV-Strahlungsquelle (24, 24a, 24b) in Abstrahlungsrichtung (R) weiter entfernte Behandlungskammer (K2, K3, K4) mündet (26).

2. Vorrichtung nach Anspruch 1,
**dadurch gekennzeichnet, dass**
die UV-Strahlungsquelle (24; 24a, 24b) ein UV-Strahlungsspektrum von mindestens 180 nm bis zumindest 254 nm Wellenlänge besitzt.

3. Vorrichtung nach einem oder mehreren der vorhergenannten Ansprüche
**dadurch gekennzeichnet, dass**
die mehreren Schichten von Behandlungskammern (K1, K4) in radialer Richtung (R) um die UV-Strahlungsquelle (24) herum angeordnet sind.

4. Vorrichtung nach einem oder mehreren der vorhergenannten Ansprüche
**dadurch gekennzeichnet, dass**
die mehreren Schichten von Behandlungskammern (K1 - K4) konzentrisch um die UV-Strahlungsquelle (24) herum angeordnet sind.

5. Vorrichtung nach einem oder mehreren der vorhergenannten Ansprüche
**dadurch gekennzeichnet, dass**
sich mindestens eine der Behandlungskammern (K1 - K4) der mehreren Schichten von Behandlungskammern (K1 - K4) spiralförmig um die UV-Strahlungsquelle (24) herum erstreckt.

6. Vorrichtung nach einem oder mehreren der vorher genannten Ansprüche,
**dadurch gekennzeichnet, dass**
ausgehend von der UV-Strahlungsquelle (24; 24a, 24b) in Abstrahlungsrichtung (R) mindestens die erste (T1) der transparenten UV-durchlässigen Trennschichten (T1 - T4), die zwischen der UV-Strahlungsquelle (24; 24a, 24b) und der ersten Behandlungskammer (K1) angeordnet ist und der UV-Strahlungsquelle (24; 24a, 24b) am nächsten liegt, aus einem ersten Trennschicht-Material hergestellt ist, welches eine UV-Durchlässigkeit besitzt, die im Wesentlichen das gesamte UV-Strahlungsspektrum der UV-Strahlungsquelle (24; 24a, 24b) einschließlich eines ersten Teilspektrums mit Wellenlängen kleiner 200nm, insbesondere einen Wellenlängenbereich kleiner 200nm und größer gleich 180nm, durchlässt.

7. Vorrichtung nach einem oder mehreren der vorher genannten Ansprüche,
**dadurch gekennzeichnet, dass**
wenigstens eine weitere (T2) der Trennschichten (T1 - T4), welche der ersten Trennschicht (T1) in Abstrahlungsrichtung (R) nachfolgt, aus dem ersten Trennschicht-Material hergestellt ist.

8. Vorrichtung nach einem oder mehreren der vorher genannten Ansprüche,
**dadurch gekennzeichnet, dass**
die in Abstrahlungsrichtung (R) gemessene Gesamtdicke einer Teilschicht,
die aus einer oder mehreren Trennschichten (T1, T2) aus erstem Trennschicht-Material und einer oder mehreren Behandlungskammern (K1, K2) gebildet wird, in welche die UV-Strahlung durch das erste Trennschicht-Material eintritt,
im Wesentlichen einer maximalen effektiven Eindringtiefe des ersten Teilspektrums der UV-Strahlung entspricht,
die sich in Abhängigkeit der Absorptionsfähigkeit des jeweils zu behandelnden Mediums und der UV-Durchlässigkeit des ersten Trennschicht-Materials für dieses erste Teilspektrum ergibt.

9. Vorrichtung nach einem oder mehreren der vorher genannten Ansprüche,
**dadurch gekennzeichnet, dass**
mindestens eine weitere (T3, T4) der Trennschichten (T1 - T4), welche in Abstrahlungsrichtung einer Trennschicht (T1, T2) nachfolgt, die aus dem ersten Trennschicht-Material hergestellt ist, aus einem zweiten Trennschicht-Material gefertigt ist, welches eine UV-Durchlässigkeit besitzt, die von dem UV-Strahlungsspektrum der UV-Strahlungsquelle (24; 24a, 24b) nur UV-Strahlung in einem zweiten Teilspektrum mit Wellenlängen größer gleich 200nm durchlässt.

10. Vorrichtung nach einem oder mehreren der vorhergenannten Ansprüche
**dadurch gekennzeichnet, dass**
diese eine Verweilzeit-Verlängerungseinrichtung zum Verlängern der Verweilzeit des zu behandelnden Mediums im Strahlungsbereich der UV-Strahlungsquelle (24; 24a, 24b) besitzt.

11. Vorrichtung nach einem oder mehreren der vorher genannten Ansprüche,
**dadurch gekennzeichnet, dass**
die Verweilzeit-Verlängerungseinrichtung einen Einspeisungskanal (4) für das zu behandelnde Medium besitzt, der bezogen auf die Abstrahlungsrichtung (R) im Wesentlichen tangential in die erste Behandlungskammer (K1) mündet und dem einzuspeisenden Medium einen Drall verleiht.

12. Vorrichtung nach einem oder mehreren der vorher genannten Ansprüche,
**dadurch gekennzeichnet, dass**
die Verweilzeit-Verlängerungseinrichtung einen Auslasskanal (4) für das behandelte Medium besitzt, der bezogen auf die Abstrahlungsrichtung (R) im Wesentlichen tangential und/oder in Drallrichtung aus der letzten, der UV-Strahlungsquelle (24) in Abstrahlungsrichtung (R) am weitesten entfernten Behandlungskammer (K4) heraustritt.

13. Vorrichtung nach einem oder mehreren der vorher genannten Ansprüche,
**dadurch gekennzeichnet, dass**
die Verweilzeit-Verlängerungseinrichtung Verwirbelungselemente (28) aufweist, die in Vorrichtungskomponenten angeordnet sind, die ausgewählt sind aus einer Gruppe von Vorrichtungskomponenten, umfassend: die Behandlungskammern (K1 - K4), den Einspeisungskanal (4), den Auslasskanal (6), Eintritts- oder Austrittsöffnungen (26) zwischen aufeinanderfolgenden Behandlungskammern (K1 - K4).

14. Vorrichtung nach einem oder mehreren der vorher genannten Ansprüche,
**dadurch gekennzeichnet, dass**
die aufeinanderfolgenden Behandlungskammern (K1 - K4) einen Teil der Verweilzeit-Verlängerungseinrichtung bilden.

15. Vorrichtung nach einem oder mehreren der vorher genannten Ansprüche,
**dadurch gekennzeichnet, dass**
die letzte der Behandlungskammern (K4) an ihrer von der UV-Strahlungsquelle in Abstrahlungsrichtung am weitesten entfernten Seite eine UV-Reflektionseinrichtung (30; 32) besitzt.

16. Verwendung der Vorrichtung nach einem oder mehreren der Ansprüche 1 bis 15 zur kombinierten UV-Entkeimung und UV-Oxidation, insbesondere zur Wasseraufbereitung.
